# EUROPÄISCHE PATENTSCHRIFT

(11) **EP 0 893 426 B1**
(45) Veröffentlichungstag und Bekanntmachung des Hinweises auf die Patenterteilung: **31.10.2001**
(21) Anmeldenummer: 98113363.0
(22) Anmeldetag: 17.07.1998
(51) Int. Cl.: C07C 67/14

(54) **2-Phasen-Herstellung von Carbonsäureestern**
Biphasic process for preparing carboxylic esters
Procédé biphasique pour la préparation d'esters carboxyliques

(30) Priorität: 25.07.1997 DE 19732031
(43) Veröffentlichungstag der Anmeldung: 27.01.1999
(73) Patentinhaber: Solvay Fluor und Derivate GmbH, 30173 Hannover (DE); Braun, Max, Dr., 30900 Wedemark (DE)
(72) Erfinder: Braun, Max, 30900 Wedemark (DE); Eichholz, Kerstin, 30855 Langenhagen (DE); Rudolph, Werner, 30559 Hannover (DE)
(74) Vertreter: Lauer, Dieter, Dr.

(56) Entgegenhaltungen:
- EP-A- 0 209 157
- EP-A- 0 623 582
- DE-A- 4 313 793

## Beschreibung

Die Erfindung bezieht sich auf ein Verfahren zur katalysierten Herstellung von Methyl- und Ethylestern der Trifluor- und Chlordifluoressigsäure.

Die obengenannten Ester der Trifluor- bzw. Chlordifluoressigsäure können als Lösungs- oder Reinigungsmittel eingesetzt werden. So ist der Trifluoressigsäureethylester ein Lösungsmittel bei der Paraffinchlorierung. Die Ester sind auch Zwischenprodukte in der chemischen Synthese. Der Trifluoressigsäuremethylester und der Trifluoressigsäure-1.1.1-Trifluorethylester liefern nach Hydrierung Trifluorethanol (und gegebenenfalls Methanol). Trifluorethanol ist ein Lösungsmittel und auch ein Zwischenprodukt für die Herstellung von Isoflurane, ein Anästhetikum. Der Methyl- bzw. Ethylester der Chlordifluoressigsäure sind ebenfalls Synthesebausteine, beispielsweise bei der Herstellung von Flüssigkristallen.

Das US-Patent 5,405,991 (= EP-A-0 623,582) offenbart die Herstellung von Estern der Trifluoressigsäure und der Chlordifluoressigsäure aus den Säurechloriden und dem entsprechenden Alkohol in Anwesenheit von Alkalimetall- oder "Onium"-Salzen der dem eingesetzten Carbonsäurechlorid entsprechenden Carbonsäure.

Aufgabe der vorliegenden Erfindung ist es, im wesentlichen reine Ester (Reinheitsgrad >94 Gew.-%) auf noch einfachere Weise zu erhalten. Diese Aufgabe wird durch das in den Ansprüchen angegebene Verfahren gelöst.

Die Erfindung beruht auf der Beobachtung, daß bei der Herstellung der Methyl- bzw. Ethylester der Trifluoressigsäure bzw. Chlordifluoressigsäure aus den Säurechloriden und dem Alkohol in Anwesenheit von "Onium"-Salzen der betreffenden Säuren als Katalysator sich unter bestimmten Molverhältnissen zwei Phasen ausbilden, und zwar derart, daß im 2-Phasen-Bereich stets eine Phase den im wesentlichen reinen Ester umfaßt.

Das erfindungsgemäße Verfahren zur Herstellung von Methyl- oder Ethylestern der Trifluoressigsäure und der Chlordifluoressigsäure sieht vor, daß man das Säurechlorid mit einem Überschuß des Alkohols in Anwesenheit eines Onium-Salzes der betreffenden Säure umsetzt und man das Molverhältnis von Alkohol zu Säurechlorid so wählt, daß sich zwei Phasen bilden, wobei eine Phase den Ester in einer, ohne Destillationsstufe erzielbaren Reinheit von mindestens 95 Gew.-% enthält. Der Ester kann durch Abtrennen der Esterphase von der anderen Phase isoliert werden. Bei dieser Vorgehensweise fällt der Ester somit in einer Reinheit an, die eine Destillation erübrigt. Eine bevorzugte Ausführungsform des erfindungsgemäßen Verfahrens sieht deshalb die Isolierung des erhaltenen Esters ohne Destillation vor. Das Molverhältnis von Alkohol zu Säurechlorid liegt vorteilhaft im Bereich von 1,01:1 bis 5:1.

Bei der Herstellung des Methylesters der Trifluoressigsäure liegt das Molverhältnis von Methanol zu Trifluoracetylchlorid im Bereich von 1,03:1 bis 4:1. Bei der Herstellung des Ethylesters der Trifluoressigsäure liegt das Molverhältnis von Ethanol zu Trifluoracetylchlorid im Bereich von 1,01:1 bis 5:1. Bei der Herstellung des Methylesters der Chlordifluoressigsäure liegt das Molverhältnis von Methanol zu Chlordifluoracetylchlorid im Bereich von 1,06:1 bis 2,5:1. Bei der Herstellung des Ethylesters der Chlordifluoressigsäure liegt das Molverhältnis von Ethanol zu Chlordifluoracetylchlorid im Bereich von 1,02:1 bis 2,5:1. In den genannten Bereichen liegen zwei Phasen vor, in welchen wie gesagt eine Phase den Ester umfaßt, der stets in einer Reinheit von mindestens 95 Gew.-% enthalten ist. Die Methylester bilden stets die untere Phase; der Ethylester der Chlordifluoressigsäure bildet ebenfalls die untere Phase, der Ethylester der Trifluoressigsäure bildet die obere Phase.

Wie beim gattungsgemäßen Verfahren ist ein Zusatz einer Säure nicht notwendig und erfolgt vorzugsweise nicht.

Vorzugsweise wird das Verfahren kontinuierlich durchgeführt.

Der Begriff "Onium" steht für Kationen mit positiv geladenem Stickstoff, beispielsweise protonierte aromatische Stickstoffbasen wie Pyridinium oder protonierte Alkyl-, Dialkyl- oder Trialkylammonium-Kationen mit bis zu 20 C-Atomen, oder für durch Cycloalkyl substituierte Ammonium-Verbindungen oder cycloaliphatische Stickstoffbasen wie Piperidinium oder quartäre Ammonium-Kationen.

Sehr gut geeignet als Carbonsäuresalz sind "Onium"-Salze, wobei "Onium" für ein Kation des Stickstoffs der Formel R'R"R"'R""N⁺, worin R,'R",R"' und R"" unabhängig voneinander für Wasserstoff, Alkyl mit 1 bis 20 C-Atomen, Aryl oder Aralkyl stehen, oder worin R' und R" oder worin R"' und R"", oder worin R', R" und R"' oder worin R', R", R"' und R"", gegebenenfalls unter Einschluß des Stickstoff-Atoms, gesättigte oder ungesättigte Ringsysteme bilden. "Aryl" bedeutet hier insbesondere Phenyl oder durch 1 oder mehrere C1-C2-Alkylgruppen substituiertes Phenyl. Hervorragend geeignet sind Salze, in denen "Onium" für Ammonium, Pyridinium oder R¹'R²'R³'R⁴'N⁺ steht, worin R¹', R²', R³' und R⁴' unabhängig voneinander Wasserstoff, Alkyl mit 1 bis 15 C-Atomen, Phenyl oder Benzyl stehen. Als Beispiel für solche Kationen seien genannt Pyridinium, Piperidinium, Anilinium, Benzyltriethylammonium und Triethylammonium.

Die Temperatur, bei der die Umsetzung durchgeführt wird, liegt bei Umgebungstemperatur (etwa 20 °C) bis hin zum Siedepunkt der Mischung, beispielsweise bis hin zu 100 °C. Man arbeitet bei Umgebungsdruck (etwa 1 bar abs.) oder gewünschtenfalls auch bei erhöhtem Druck, beispielsweise bei einem Druck von bis zu 5 bar abs.

Das "Onium"-Salz kann in katalytischen oder molaren Mengen anwesend sein. Zweckmäßig liegt das Mol-Verhältnis zwischen dem Säurehalogenid und dem Carbonsäuresalz (Oniumsalz) im Bereich von 1:1 und 20:1, aber man kann auch außerhalb dieses Bereiches noch arbeiten. Erstaunlicherweise führt die Anwesenheit des Oniumsalzes zu höherer Reinheit der Esterphase. Die optimale Menge an Oniumsalz für die Erreichung einer gewünschten Reinheit kann man durch Handversuche leicht ermitteln - einfach indem man die Esterphase jeweils analysiert, z. B. durch Gaschromatogramm.

Gemäß einer besonderen Ausführungsform der Erfindung erzeugt man das Säurechlorid und das "Onium"-Salz der Carbonsäure in situ. Hierzu setzt man das entsprechende "Onium"-Chlorid mit dem Anhydrid der einzusetzenden Carbonsäure um. Bei dieser Umsetzung bildet sich aus dem Anhydrid der Carbonsäure das entsprechende Säurehalogenid und das entsprechende Salz. Bei dieser Ausführungsform kann man als Alkalimetall-oder "Onium"-Halogenid verbrauchte Halogenid-Katalysatoren einsetzen, die auf diese Weise in Wertprodukte überführt werden können.

Vorzugsweise verwendet man Pyridinium-Salze.

Die Erfindung weist den Vorteil auf, daß man ohne hydrolytische Aufarbeitung in technisch besonders einfacher, energiesparender Weise Carbonsäureester erzeugen kann.

Die Erfindung wird anhand der folgenden Beispiele weiter erläutert, ohne daß sie in ihrem Umfang eingeschränkt werden soll.

### Herstellung von Trifluoressigsäurealkylestern

### Beispiel 1:

### Trifluoressigsäureethylester

| Ansatz: | |
|---|---|
| 0,3 mol Pyridin | 23,7 g |
| 0,3 mol Trifluoressigsäure | 34,2 g |
| 3,0 mol Ethanol p.A. | 138,3 g |
| 2,7 mol Trifluoracetylchlorid | 357,7 g |

### Durchführung:

In einem 500 ml Dreihalskolben mit Magnetrührstab, Thermofühler und Trockeneiskühler wird das Pyridin vorgelegt und TFA zugetropft. Die Reaktion ist exotherm. Bevor nun das Pyridiniumtrifluoracetat kristallisiert, wird das Ethanol zugegeben, um es in Lösung zu halten. Die Reaktionsmischung wird im Ölbad auf 50 °C vortemperiert und bei dieser Temperatur das TFAC über eine Glasfritte eingeleitet. Bei 20 % der zum Ethanol stöchiometrischen TFAC-Menge bilden sich zwei Phasen, wobei die obere Phase nahezu reiner Trifluoressigsäureethylester ist. Nach Beendigung des TFAC-Einleitens (2,7 mol, 90 % der Stöchiometrie) wird die Reaktionsmischung in einen Scheidetrichter überführt. Beide Phasen sind klar, die Katalysatorphase ist leicht gelb gefärbt. Die Analytik der Esterphase erfolgte mittels Gaschromatographie und ergab Ester mit 97 % Reinheit. Die in diesem Versuch isolierte Esterausbeute betrug 96,6 % der Theorie. Die Probenahme aus der Esterphase im Zweiphasenbereich bei 50, 60, 70, 80 und 90 % der stöchiometrischen TFAC-Menge ergab jeweils vergleichbare Esterreinheiten.
Das Zweiphasengebiet Esterphase/Katalysatorphase wird erst bei 99 % der TFAC/Alkohol Stöchiometrie verlassen.

### Beispiel 2:

### Trifluoressigsäuremethylester

| Ansatz: | |
|---|---|
| 0,3 mol Pyridin | 23,7 g |
| 0,3 mol Trifluoressigsäure (TFA) | 34,2 g |
| 3,0 mol Methanol | 96,12 g |
| 2,7 mol Trifluoracetylchlorid (TFAC) | 357,70 g |

### Durchführung:

In einem 500 ml Dreihalskolben mit Magnetrührstab, Thermofühler und Trockeneiskühler wird das Pyridin vorgelegt und TFA zugetropft. Die Reaktion ist exotherm. Bevor nun das Pyridiniumtrifluoracetat kristallisiert, wird das Methanol zugegeben, um es in Lösung zu halten. Die Reaktionsmischung wird im Ölbad auf 50 °C vortemperiert und bei dieser Temperatur das TFAC über eine Glasfritte eingeleitet.

Bei 25 % des benötigten TFAC's bilden sich zwei Phasen, wobei die untere Phase fast reiner Trifluoressigsäuremethylester ist. Die Temperatur sinkt im Lauf der Reaktion auf die Siedetemperatur des Esters ab. Die Reaktionsdauer beträgt 3 Stunden. Die Analytik der Esterphase erfolgte mittels Gaschromatographie und ergab 98,5 % Ester, die isolierte Esterausbeute betrug 95,2 % der Theorie. Die Probenahme aus der Esterphase im Zweiphasenbereich bei 50, 60, 70, 80 und 90 % der stöchiometrischen TFAC-Menge ergab jeweils vergleichbare Esterreinheiten. Das Zweiphasengebiet Esterphase/Katalysatorphase wird erst bei 97 % der TFAC/Alkohol Stöchiometrie verlassen.

### Herstellung von Chlordifluoressigsäurealkylestern

### Beispiel 3:

### Chlordifluoressigsäuremethylester

| Ansatz: | |
|---|---|
| 0,2 mol Pyridin | 23,7 g |
| 0,19 mol Chlordifluoressigsäure (CDFA) | 34,2 g |
| 2,0 mol Methanol p.A. | 138,3 g |
| 1,8 mol Chlordifluoracetylchlorid (CDFAC) | 268,06 g |

### Durchführung:

In einem 500 ml Dreihalskolben mit Magnetrührstab, Thermofühler und Trockeneiskühler wird das Pyridin vorgelegt und CDFA zugetropft. Die Reaktion ist exotherm. Bevor nun das Pyridiniumchlordifluoracetat kristallisiert, wird das Methanol zugegeben, um es in Lösung zu halten. Die Reaktionsmischung wird im Ölbad auf 50 °C vortemperiert und bei dieser Temperatur das CDFAC über den Tropftrichter eingeleitet.

Bei ca. 40 % der zum Alkohol stöchiometrischen CDFAC-Menge bilden sich zwei Phasen, wobei die untere Phase fast reiner Chlordifluoressigsäuermethylester ist. Nach Beendigung des Zutropfens von 1,8 mol CDFAC wird kurz nachgerührt und anschließend die Phasen getrennt. Die Phasen sind nach der Trennung klar, die Katalysatorphase ist dunkelgelb gefärbt. Die Esterphase wurde mittels Gaschromatographie untersucht und ergab eine Chlordifluoressigsäuremethylesterreinheit von 95 %.

Das Zweiphasengebiet Esterphase/Katalysatorphase wird erst bei ca. 94 % der CDFAC/Alkohol Stöchiometrie verlassen.

### Beispiel 4:

### Chlordifluoressigsäureethylester

| Ansatz: | |
|---|---|
| 0,2 mol Pyridin | 23,7 g |
| 0,19 mol Chlordifluoressigsäure (CDFA) | 34,2 g |
| 2,0 mol Ethanol p.A. | 92,12 g |
| 1,8 mol Chlordifluoracetylchlorid (CDFAC) | 268,06 g |

### Aufbau u. Durchführung:

In einem 500 ml Dreihalskolben mit Magnetrührstab, Thermofühler und Trockeneiskühler wird das Pyridin vorgelegt und CDFA zugetropft. Die Reaktion ist exotherm. Bevor nun das Pyridiniumchlordifluoracetat kristallisiert, wird das Ethanol zugegeben, um es in Lösung zu halten. Die Reaktionsmischung wird im Ölbad auf 50 °C vortemperiert und bei dieser Temperatur CDFAC aus einem Tropftrichter zugetropft.

Bei ca. 40 % der zum Alkohol stöchiometrischen CDFAC-Menge bilden sich zwei Phasen, wobei die untere Phase fast reiner Chlordifluoressigsäueethylester ist. Nach Beendigung des Zutropfens von 1,8 mol CDFAC wird kurz nachgerührt und anschließend die Phasen getrennt.

Die Phasen sind nach der Trennung klar, die Katalysatorphase ist dunkelgelb gefärbt.

Die Analytik der Esterphase ergab eine Ester-Reinheit von 96,5 %.

Das 2-Phasen-Gebiet wird erst bei ca. 98 % der CDFAC/Alkohol-Stöchiometrie verlassen.

Das Onium-Salz konnte nach der Abtrennung der Esterphase mehrfach erneut als Katalysator für die erfindungsgemäße Veresterungsreaktion eingesetzt werden. Somit ist das Verfahren auch kontinuierlich durchführbar.

## Patentansprüche

1. Verfahren zur Herstellung von Methyl- oder Ethylestern der Trifluoressigsäure und der Chlordifluoressigsäure, wobei man das Säurechlorid mit einem stöchiometrischen Überschuß des Alkohols in Anwesenheit eines Oniumsalzes der Säure als Katalysator umsetzt und man das Molverhältnis von Alkohol zu Säurechlorid so wählt, daß sich zwei Phasen bilden, wobei eine Phase den Ester in einer, ohne Destillationsstufe erzielbaren Reinheit von mindestens 95 Gew.-% enthält, wobei, wenn man den Methylester der Trifluoressigsäure herstellt, das Molverhältnis von Methanol zu Trifluoracetylchlorid im Bereich von 1,03:1 bis 4:1 liegt, oder, wenn man den Ethylester der Trifluoressigsäure herstellt, das Molverhältnis von Ethanol zu Trifluoracetylchlorid im Bereich von 1,01:1 bis 5:1 liegt, oder, wenn man den Methylester von Chlordifluoressigsäure herstellt, das Molverhältnis von Methanol zu Chlordifluoracetylchlorid im Bereich von 1,06:1 bis 2,5:1 liegt, oder, wenn man den Ethylester der Chlordifluoressigsäure herstellt, das Molverhältnis von Ethanol zu Chlordifluoracetylchlorid im Bereich von 1,02:1 bis 2,5:1 liegt.

2. Verfahren nach Anspruch 1, **dadurch gekennzeichnet, daß** man den Ester durch Abtrennen der Esterphase von der anderen Phase isoliert.

3. Verfahren nach Anspruch 1, **dadurch gekennzeichnet, daß** man es kontinuierlich durchführt.

4. Verfahren nach Anspruch 1, **dadurch gekennzeichnet, daß** man das Pyridiniumsalz der betreffenden Säure als Katalysator einsetzt.

## Claims

1. A method for the preparation of methyl or ethyl esters of trifluoroacetic acid and of chlorodifluoroacetic acid, in which the acid chloride is reacted with a stoichiometric excess of the alcohol in the presence of an onium salt of the acid as catalyst and the molar ratio of alcohol to acid chloride is selected such that two phases are formed, one phase containing the ester in a purity, achievable without a distillation stage, of at least 95% by weight, wherein when the methyl ester of trifluoroacetic acid is prepared the molar ratio of methanol to trifluoroacetyl chloride is in the range from 1.03:1 to 4:1, or, when the ethyl ester of trifluoroacetic acid is prepared, the molar ratio of ethanol to trifluoroacetyl chloride is in the range from 1.01:1 to 5:1, or, when the methyl ester of chlorodifluoroacetic acid is prepared, the molar ratio of methanol to chlorodifluoroacetyl chloride is in the range from 1.06:1 to 2.5:1, or, when the ethyl ester of chlorodifluoroacetic acid is prepared, the molar ratio of ethanol to chlorodifluoroacetyl chloride is in the range from 1.02:1 to 2.5:1.

2. A method according to Claim 1, **characterised in that** the ester is isolated by separating the ester phase from the other phase.

3. A method according to Claim 1, **characterised in that** it is performed continuously.

4. A method according to Claim 1, **characterised in that** the pyridinium salt of the acid in question is used as catalyst.

## Revendications

1. Procédé de préparation d'esters méthylique ou éthylique de l'acide trifluoroacétique et de l'acide chlorodifluoroacétique, dans lequel on fait réagir le chlorure d'acide avec un excès stoechiométrique de l'alcool en présence d'un sel d'onium de l'acide comme catalyseur et on choisit le rapport molaire de l'alcool au chlorure d'acide de manière qu'il se forme deux phases, une phase contenant l'ester à une pureté d'au moins 95 % en poids, que l'on peut obtenir sans étape de distillation, et, lorsqu'on produit l'ester méthylique de l'acide trifluoroacétique, le rapport molaire du méthanol au chlorure de trifluoroacétyle se situe dans un intervalle de 1,03:1 à 4:1, ou, lorsqu'on produit l'ester éthylique de l'acide trifluoroacétique, le rapport molaire de l'éthanol au chlorure de trifluoroacétyle se situe dans un intervalle de-1,01:1 à 5:1, ou, lorsqu'on produit l'ester méthylique de l'acide chlorodifluoroacétique, le rapport molaire du méthanol au chlorure de chlorodifluoroacétyle se situe dans un intervalle de 1,06:1 à 2,5:1, ou lorsqu'on produit l'ester éthylique de l'acide chlorodifluoroacétique, le rapport molaire de l'éthanol au chlorure de chlorodifluoroacétyle se situe dans un intervalle de 1,02:1 à 2,5:1.

2. Procédé selon la revendication 1, **caractérisé en ce qu'**on isole l'ester par séparation de la phase ester d'avec l'autre phase.

3. Procédé selon la revendication 1, **caractérisé en ce qu'**on procède de façon continue.

4. Procédé selon la revendication 1, **caractérisé en ce qu'**on utilise le sel de pyridinium de l'acide correspondant comme catalyseur.
